(19) Europäisches Patentamt

European Patent Office

Office européen des brevets

(11) Numéro de publication: **0 265 338 B1**

(12) **FASCICULE DE BREVET EUROPEEN**

(45) Date de publication de fascicule du brevet: **06.05.92**  (51) Int. Cl.⁵: **A61K 35/78**, A61K 47/00

(21) Numéro de dépôt: **87402345.0**

(22) Date de dépôt: **20.10.87**

Le dossier contient des informations techniques présentées postérieurement au dépôt de la demande et ne figurant pas dans le présent fascicule.

(54) **Compositions thérapeutiques stables à base d'huile végétale hydrophobe et son procédé de préparation.**

(30) Priorité: **20.10.86 FR 8614544**

(43) Date de publication de la demande:
**27.04.88 Bulletin 88/17**

(45) Mention de la délivrance du brevet:
**06.05.92 Bulletin 92/19**

(84) Etats contractants désignés:
**AT BE CH DE ES FR GB GR IT LI LU NL SE**

(56) Documents cités:
**EP-A- 0 107 085**
**FR-A- 2 556 968**

(73) Titulaire: **PIERRE FABRE MEDICAMENT**
**45, Place Abel Gance**
**F-92100 Boulogne(FR)**

(72) Inventeur: **Dupinay, Pierre**
**4, rue de la République**
**F-81200 Mazamet(FR)**
Inventeur: **Bauer, Michel**
**150, Chemin des Fourches Hautes**
**F-81100 Castres(FR)**

(74) Mandataire: **Warcoin, Jacques et al**
**Cabinet Régimbeau 26, avenue Kléber**
**F-75116 Paris(FR)**

Rank Xerox (UK) Business Services
(3.08/2.18/2.0)

## Description

La présente invention concerne le domaine des compositions thérapeutiques comportant un principe actif insoluble dans l'eau, ce principe actif pouvant être solide, pâteux ou liquide.

L'efficacité d'un médicament est étroitement dépendante de la cinétique de dissolution de la substance active qui y est contenue. Or, il arrive souvent qu'un principe actif, dont l'efficacité ou le champ d'action sont très prometteurs, est mal exploité du fait d'un caractère hydrophobe trop important empêchant ou ralentissant considérablement sa dissolution. Certains principes actifs sont même laissés hors du circuit commercial dans l'attente d'une découverte permettant ou améliorant leur dissolution.

Il existe en particulier un grand nombre d'huiles végétales hydrophobes dont on connaît ou dont on soupçonne l'activité, mais dont la mauvaise cinétique de dissolution empêche leur exploitation.

La présente invention a permis de remédier à ces lacunes en proposant une nouvelle composition thérapeutique comportant un principe actif insoluble dans l'eau, plus particulièrement une huile vegétale hydrophobe, auquel on a associé un ou plusieurs polyoxyéthylèneglycols (PEG).

Cette association permet, non seulement l'amélioration de la vitesse de dissolution du principe actif lors de son administration per os, mais aussi l'obtention d'une composition médicamenteuse stable dans le temps et à la chaleur. Cette stabilité permet d'éviter, au cours du stockage, des pertes par fuites du mélange PEG-principe actif ou par exsudation du principe actif seul.

En outre, les PEG présentent une bonne tolérance digestive, une totale inocuité et surtout respectent l'intégrité du principe actif.

Le principe actif, choisi de préférence dans le cadre dela présente invention, est un extrait lipidostérolique de Serenoa repens. Le Serenoa repens ou Sabal serrulatum, est un petit palmier que l'on rencontre sur des terrains sablonneux, arides et incultes.

La composition de l'extrait lipidostérolique de Serenoa repens utilisé dans le cadre de la présente invention, est la suivante

a) Cet extrait comporte :

pour la partie saponifiable :

- de l'acide laurique (environ 25 % en poids)
- de l'acide myristique (environ 10 % en poids)
- de l'acide palmitique (environ 10 % en poids)
- de l'acide oléique (environ 35 % en poids)

b) pour la partie insaponifiable :

- des alcools gras (hexacosanol, octacosanol, triacontanol)
- du $\beta$-stigmastérol, du campestérol, du stimagstérol, et du cycloarténol.

La demande FR A 2 556 968, déposée par le Demandeur décrit des compositions dermatologiques à base d'une fraction lipidique extraite de fruits Serenoa repens.

La présente invention vise notamment à améliorer la cinétique de dissolution d'un tel extrait en associant ce dernier avec un ou plusieurs PEG.

On utilise de préférence des PEG de poids moléculaire moyens ou élevés et notamment les PEG 6000 (poids moléculaire compris entre 5600 et 7000), les PEG 10000 (poids moléculaire compris entre 8500 et 12500) ou les PEG 20000 (poids moléculaire d'environ 20000).

Ces PEG peuvent être utilisés seuls ou en mélange entre eux, suivant le principe actif utilisé, sa quantité et les résultats que l'on désire obtenir. L'association de différents PEG permet d'obtenir des PEG de poids moléculaires intermédiaires tels que des PEG 8000 ou des PEG 16000.

La composition préférée selon la présente invention comporte, en poids :

- 10 à 40 % d'un extrait lipido-stérolique de Serenoa repens,
- 60 à 90 % d'un ou plusieurs PEG.

Ainsi, par exemple, une composition comportant 160 mg d'extrait et 290 mg de PEG correspond à un dosage unitaire idéal.

Diverses formes galéniques peuvent être utilisées pour l'administration par voie orale de la composition selon l'invention. Les capsules constituent cependant, la forme galénique la plus appropriée et, parmi elles, la gélule est la forme préférée dans la mesure où sa petite taille permet un temps de désagrégation rapide. De plus, la gélule permet aussi bien l'administration de mélanges poudreux que l'administration de mélanges pâteux.

Enfin, la gélule peut être fabriquée sur du matériel industriel automatique d'utilisation courante.

Ainsi, la formulation préférée selon l'invention correspond à une gélule de 450 mg répondant à la composition suivante :

- Extrait lipido stérolique de Serenoa repens          160 mg
- Polyoxyéthylèneglycol 10000          290 mg

Ces proportions excipient - principe actif permettent en effet, dans une gélule de taille raisonable :

- une désagrégation très rapide de la gélule et du mélange qu'elle contient,

- une vitesse de libération du principe actif supérieure à celle du principe actif seul placé dans les mêmes conditions;
- l'absence d'exsudation du principe actif dans le temps et à la chaleur (45°C),
- l'absence de fluidification du mélange dans le temps et à la chaleur (45°C) et l'absence de fuites entre corps et tête de la gélule.

En effet, au cours du temps, on n'observe pas de démixtion, ni de fuite de principe actif après 1 an à 45°C.

De plus, dans les milieux de délitement et de dissolution, on observe une très bonne dispersion du mélange (solutions opalescentes) sans remontée du principe actif en surface sous forme d'huile.

Enfin, les analyses effectuées par détermination pondérale et par spectroscopie infrarouge montrent que le principe actif n'est pas modifié par l'excipient et ne forme pas d'ester ou de complexe avec le ou les PEG.

Le mélange décrit ci-dessus, se caractérise aussi par un point de fusion peu élevé, et sa solidification rapide. Cette propriété sera fort utile pour son procédé de préparation.

En effet, la composition, selon la présente invention, pourra être préparée par différents procédés connus en la matière. Mais de préférence, on effectuera le mélange du principe actif et du ou des PEG par cofusion. La dispersion du principe actif dans le ou les PEG sera ainsi facile, homogène et durable.

Le point de fusion peu élevé des PEG permet de respecter la stabilité du principe actif ainsi que l'enveloppe de la gélule. De plus, les PEG donnent au mélange à chaud (55°C) une rhéologie adéquate (ni trop fluide, ni trop épaisse) de manière à faciliter la mise en gélule et assurer un bon dosage unitaire tout au long du remplissage.

Le mélange une fois en gélules devra se solidifier rapidement afin d'éviter les pertes par écoulement hors de la gélule entre tête et corps.

Quant à l'encapsulation proprement dite, elle se fera selon les procédés connus de l'homme de l'art.

Du fait de la stabilité à la chaleur de la composition selon l'invention, on pourra, en particulier, opérer par thermofusion.

La présente invention est illustrée par l'exemple et les figures suivantes :

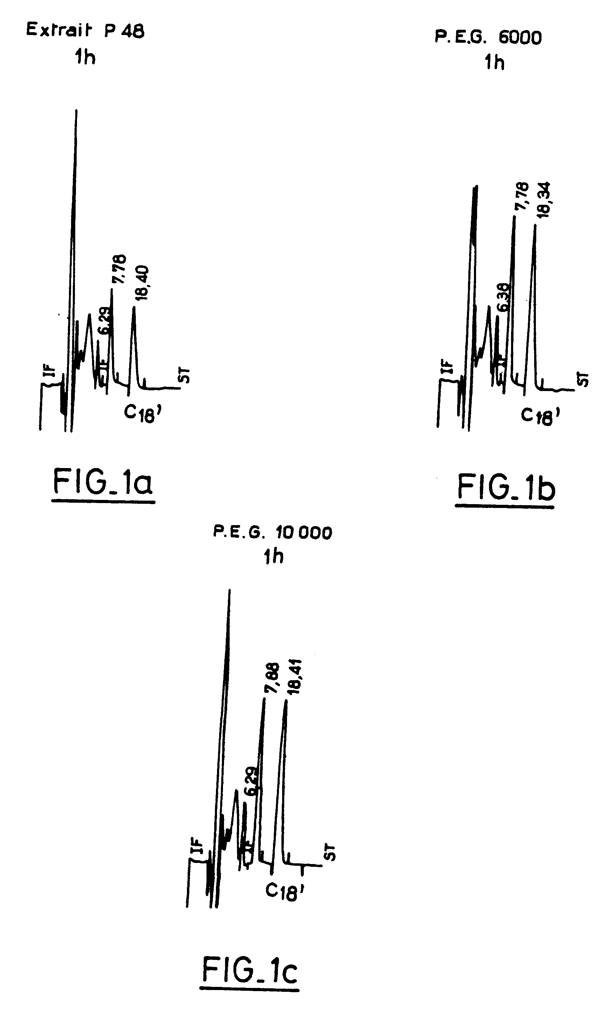

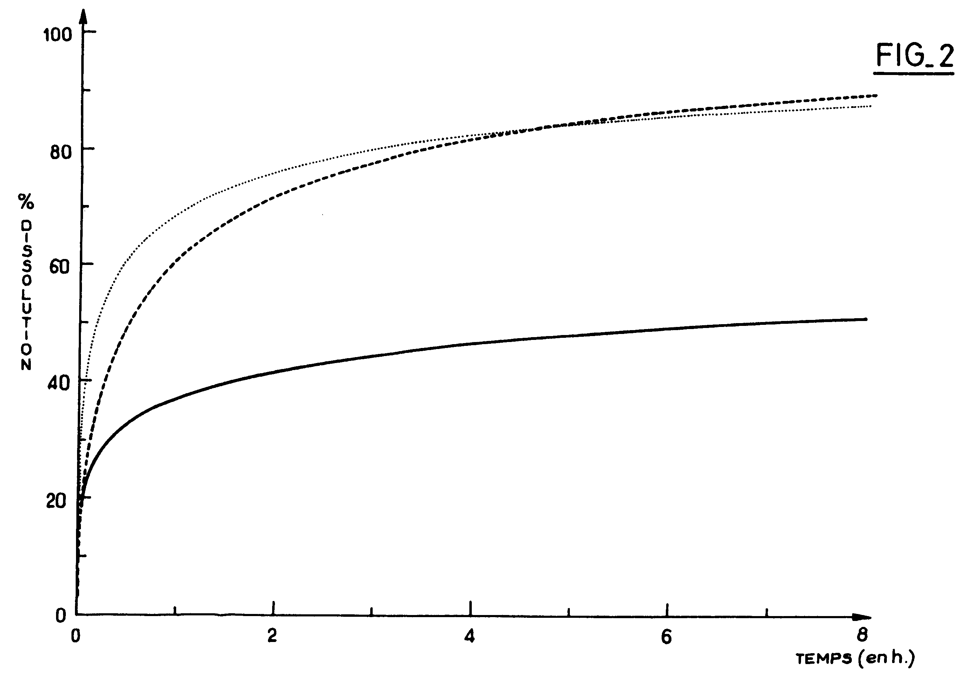

- . La Figure 1a représente le chromatogramme obtenu dans l'essai de dissolution de l'extrait seul ;
- . La figure 1b représente le chromatogramme obtenu dans l'essai de dissolution de l'extrait à partir des gélules remplies par du PEG 6000 ;
- . La figure 1C représente le chromatogramme obtenu dans l'essai de dissolution de l'extrait à partir des gélules remplies par du PEG 10000 ;
- . La figure 2 représente le pourcentage de dissolution par rapport à la quantité théorique de l'extrait P48 seul (——) ; l'extrait P48 contenu dans des gélules remplies de PEG 6000 (.....) ; l'extrait P48 contenu dans des gélules remplies de PEG 10000 (-----)

EXEMPLE

ETUDE ANALYTIQUE COMPAREE DE LA DISSOLUTION DE L'EXTRAIT LIPIDO STEROLIQUE DE SERENOA REPENS (P48) SEUL ET A PARTIR DES FORMULATIONS PATEUSES DECRITES

Appareillage :

Appareil type POOLE à palette tournante thermostatée à 37°C avec une vitesse de rotation fixée à 100 tours/min.

Milieu de dissolution :

Tampon phosphate pH = 7,5[(*)]        85 ml
Méthanol        15 ml

Méthode de dosage :

La dissolution de l'extrait (P48) est suivi par le dosage par chromatographie liquide haute performance de l'acide oléique ($C_{18}$) composant majoritaire de l'extrait.

Appareillage :

- . chromatographe type WATERS ou équivalent muni d'un détecteur à longueur d'onde variable, fixé ici à 205 nm et d'un injecteur automatique type WISP.
- . Colonne en acier inoxydable, de longueur : 30 cm et diamètre interne : 4,2 mm remplie par de la phase Microbondapack®      -($C_{18}$-10 $\mu$m).
- . Eluant :
  Acétonitrile        900 ml
  Solution de phosphate monopotassique ($KH_2PO_4$) 0,001 M        100 ml
  Acide phosphorique à 85 %        0,1 ml
- . Débit : 1 ml/mn
- . Injection : 20 $\mu$l

(*) le tampon est préparé de la façon suivante :
$PO_4 H_2 K$        6,8 g
Eau        900 ml
Soude concentré q.s.p. pH 7,5
Eau        q.s.p. 1000 ml

Après thermostatisation du milieu de dissolution, les gélules sont introduites directement, et l'extrait P 48 est introduit sous forme d'une dispersion aqueuse (160 mg d'extrait dans 1 ml).

Les prélèvements sont effectués à l'aide d'une seringue munie d'une membrane filtrante de 5 $\mu$m et directement injectés dans le chromatographe.

Les figures (1a), (1b) et (1c) montrent les chromatogrammes typiques obtenus dans l'étude comparée de la dissolution de l'extrait P 48.

La figure (2) montre le profil de dissolution obtenu pour :
- l'extrait P 48 seul
- l'extrait P 48 contenu dans des gélules remplies de PEG 6000
- l'extrait P 48 contenu dans des gélules remplies de PEG 10000.

Les 100 % de dissolution correspondent à la teneur théorique en extrait P 48 par gélule (160 mg).

Les courbes obtenues démontrent l'effet bénéfique des PEG sur la dissolution de l'extrait P48.

## Revendications

**Revendications pour les Etats contractants suivants : AT, BE, CH, DE, FR, GB, IT, LI, LU, NL, SE**

1. Composition thérapeutique stable, caractérisée en ce qu'elle comporte, en poids :

    10 à 40 % d'un extrait lipido-stérolique de Serenoa repens, et

    60 à 90 % d'un ou plusieurs polyoxyéthylèneglycols choisis parmi les PEG 6000, PEG 10000 et PEG 20000 ainsi que leurs mélanges.

2. Composition selon la revendication 1, caractérisée en ce qu'elle comporte :

    160 mg d'extrait lipido-stérolique de Serenoa repens, et

    290 mg de PEG 10000.

3. Composition selon l'une des revendications 1 et 2, caractérisée en ce qu'elle est conditionnée sous une forme galénique solide administrable par voie orale.

4. Composition selon la revendication 3, caractérisée en ce que la forme galénique est une capsule.

5. Composition selon la revendication 4, caractérisée en ce que la forme galénique est une gélule.

6. Procédé de préparation d'une composition selon l'une des revendications 1 à 5, caractérisé en ce que le mélange entre le principe actif et le ou les PEG est obtenu par cofusion.

**Revendications pour les Etats contractants suivants : ES, GR**

1. Procédé de préparation d'une composition thérapeutique stable, caractérisé en ce qu'on mélange :
    - 10 à 40 % en poids d'un extrait lipido-stérolique de Serenoa repens, et
    - 60 à 90 % en poids d'un ou plusieurs polyoxyéthylèneglycols choisis parmi les PEG 6000, PEG 10000 et PEG 20000 ainsi que leurs mélanges.

2. Procédé de préparation d'une composition selon la revendication 1, caractérisé en ce que ladite composition comporte :
    - 160 mg d'extrait lipido-stérolique de Serenoa repens, et
    - 290 mg de PEG 10000.

3. Procédé de préparation d'une composition selon l'une des revendications 1 et 2, caractérisé en ce que ladite composition est conditionnée sous une forme galénique solide administrable par voie orale.

4. Procédé de préparation d'une composition selon la revendication 3, caractérisé en ce que la forme galénique est une capsule.

5. Procédé de préparation d'une composition selon la revendication 4, caractérisé en ce que la forme galénique est une gélule.

6. Procédé de préparation d'une composition selon l'une des revendications 1 à 5, caractérisé en ce que le mélange entre le principe actif et le ou les PEG est obtenu par cofusion.

## Claims

**Claims for the following Contracting States : AT, BE, CH, DE, FR, GB, IT, LI, LU, NL, SE**

1. A stable therapeutic composition, characterised in that it contains the following by weight:

    10 to 40% by weight of a lipido-sterolic extract of Serenoa repens, and

    60 to 90% of one or more polyoxyethylene glycols chosen from among PEG 6000, PEG 10000 and PEG 20000, and mixtures thereof.

**2.** A composition according to claim 1, characterised in that it comprises:

160 mg of lipido-sterolic extract of Serenoa repens, and 290 mg of PEG 10000.

**3.** A composition according to claim 1 or 2, characterised in that it is packed in a galenic form for oral administration.

**4.** A composition according to claim 3, characterised in that the galenic form is a capsule.

**5.** A composition according to claim 4, characterised in that the galenic form is a gelatine capsule.

**6.** A method of preparing a composition according to any of claims 1 to 5, characterised in that the mixture between the active principle and the PEG or PEGs is obtained by cofusion.

**Claims for the following Contracting States : ES, GR**

**1.** A method of preparing a stable therapeutic composition, characterised in that the following are mixed:
- 10 to 40% by weight of a lipido-sterolic extract of Serenoa repens, and
- 60 to 90% by weight of one or more polyoxyethylene glycols chosen from among PEG 6000, PEG 10000 and PEG 20000 and mixtures thereof.

**2.** A method of preparing a composition according to claim 1, characterised in that the composition contains:
- 160 mg of lipido-sterolic extract of Serenoa repens, and
- 290 mg of PEG 10000.

**3.** A method of preparing a composition according to claim 1 or 2, characterised in that the composition is packed in a solid galenic form for oral administration.

**4.** A method of preparing a composition according to claim 3, characterised in that the galenic form is a capsule.

**5.** A method of preparing a composition according to claim 4, characterised in that the galenic form is a gelatine capsule.

**6.** A method of preparing a composition according to any of claims 1 to 5, characterised in that the mixture between the active principle and the PEG or PEGs is obtained by cofusion.

**Patentansprüche**

**Patentansprüche für folgende Vertragsstaaten : AT, BE, CH, DE, FR, GB, IT, LI, LU, NL, SE**

**1.** Therapeutisch stabile Zusammensetzung, dadurch gekennzeichnet, daß sie enthält:
10 bis 40 Gew.-% eines Lipid-Sterin-Extraktes von Serenoa repens, und 60 bis 90 Gew.-% eines oder mehrerer Polyoxyethylenglykole, ausgewählt aus PEG 6000, PEG 10 000 und PEG 20 000 sowie ihren Mischungen.

**2.** Zusammensetzung nach Anspruch 1, dadurch gekennzeichnet, daß sie enthält:
160 mg Lipid-Sterin-Extrakt von Serenoa repens und
290 mg PEG 10 000.

**3.** Zusammensetzung nach einem der Ansprüche 1 und 2, dadurch gekennzeichnet, daß sie zu einer festen galenischen Form verarbeitet ist, die auf oralem Wege verabfolgbar ist.

**4.** Zusammensetzung nach Anspruch 3, dadurch gekennzeichnet, daß die galenische Form eine Kapsel ist.

**5.** Zusammensetzung nach Anspruch 4, dadurch gekennzeichnet, daß die galenische Form eine Gelatinekapsel ist.

**6.** Verfahren zur Herstellung einer Zusammensetzung nach einem der Ansprüche 1 bis 5, dadurch gekennzeichnet, daß man die Mischung aus dem aktiven Prinzip und dem oder den PEG durch Zusammenschmelzen erhält.

**Patentansprüche für folgende Vertragsstaaten : ES, GR**

**1.** Verfahren zur Herstellung einer therapeutisch stabilen Zusammensetzung, dadurch gekennzeichnet, daß man vermischt:
10 bis 40 Gew.-% eines Lipid-Sterin-Extraktes von Serenoa repens, und 60 bis 90 Gew.-% eines oder mehrerer Polyoxyethylenglykole, ausgewählt aus PEG 6000, PEG 10 000 und PEG 20 000 sowie ihren Mischungen.

**2.** Verfahren zur Herstellung einer Zusammensetzung nach Anspruch 1, dadurch gekennzeichnet, daß die Zusammensetzung enthält:
160 mg Lipid-Sterin-Extrakt von Serenoa repens und
290 mg PEG 10 000.

3. Verfahren zur Herstellung einer Zusammensetzung nach einem der Ansprüche 1 und 2, dadurch gekennzeichnet, daß man die Zusammensetzung zu einer festen galenischen Form verarbeitet, die auf oralem Wege verabfolgbar ist.

4. Verfahren zur Herstellung einer Zusammensetzung nach Anspruch 3, dadurch gekennzeichnet, daß die galenische Form eine Kapsel ist.

5. Verfahren zur Herstellung einer Zusammensetzung nach Anspruch 4, dadurch gekennzeichnet, daß die galenische Form eine Gelatinekapsel ist.

6. Verfahren zur Herstellung einer Zusammensetzung nach einem der Ansprüche 1 bis 5, dadurch gekennzeichnet, daß man die Mischung aus dem aktiven Prinzip und dem oder den PEG durch Zusammenschmelzen erhält.

Extrait P 48
1h

FIG_1a

P.E.G. 6000
1h

FIG_1b

P.E.G. 10 000
1h

FIG_1c

FIG_2

EP 0 265 338 B1